# EUROPEAN PATENT APPLICATION

(11) **EP 3 679 931 A2**
(43) Date of publication of application: **15.07.2020**
(21) Application number: 20151676.2
(22) Date of filing: 27.04.2012
(51) Int. Cl.: A61K 31/407, A61K 38/07, A61P 25/00, A61P 25/18, A61P 25/14, A61P 25/28, A61P 3/10, A61P 9/00, A61P 25/36, A61P 25/02, A61P 25/08, A61P 25/34, A61P 25/32, A61P 25/22, A61P 27/02, A61P 27/06, A61P 29/00, A61P 25/24

(54) **METHODS OF TREATING ALZHEIMER'S DISEASE, HUNTINGTON'S DISEASE, AUTISM, AND OTHER DISORDERS**

(30) Priority: 27.04.2011 US 201161479593 P; 26.08.2011 US 201161527744 P
(62) Divisional of application: 12777016.2
(71) Applicant: Northwestern University, Evanston, IL 60208 (US)
(72) Inventor: Moskal, Joseph, Evanston, IL 60201 (US)
(74) Representative: Hoffmann Eitle

(57) **Abstract**

The disclosure relates, at least in part, to methods of treating autism in a patient in need thereof by administering an effective amount of a disclosed compound, e.g., a NMDA receptor glycine site partial agonist.

## Description

### CROSS REFERENCE TO RELATED APPLICATIONS

This application claims priority to U.S. Provisional Patent Application 61/479,593, filed April 27, 2011 and U.S. Provisional Patent Application, filed 61/527,744 filed August 26, 2011, both of which are hereby incorporated by reference in their entirety.

### BACKGROUND

Autism can be characterized by impairments in social interaction, communication, and restricted, repetitive, and stereotyped patterns of behavior. Autism is now considered to be a developmental disorder and inheritable, with presently unknown environmental exacerbating vectors. For example, twin studies show strong genetic heritability for autism; the concordance for autism is 60-91% in monozygotic twins compared to 0-10% in dizygotic twins, and 0.3% in the general population.

One key challenge in this area is the identification of autism candidate genes functionally linked to autism and could therefore be pursued as a therapeutic target. For instance, bioinformatics tools can now be used identify autism candidate genes that have been shown to have a greater number of protein-protein interactions with other autism candidate genes, called hub genes. It has been hypothesized that these hub genes can serve as "master switches" that control biological function.

Low-line animals may be used to study autism in human such as low-line rats, who engage in less social contact time with conspecifics, show lower rates of play induced pro-social ultrasonic vocalizations (USVs), and show an increased proportion of non frequency modulated (*i.e.,* monotonous), compared to non-selectively bred random-line animals.

Pharmacological-based therapeutics have had limited success and substantial side effects. Thus, behavioral modification techniques have been the most powerful positive interventions for autism spectrum disorders. The antipsychotic risperidone is the only FDA approved medication for autism, and has been reported to i) significantly decrease hyperactivity and irritability symptoms, ii) have little to no effect on inappropriate speech or social withdrawal, and iii) show significant weight gain and sedative side effects. A recent study with autistic individuals showed that D-cycloserine (DCS), an NMDAR glycine-site partial agonist, significantly improved social withdrawal (Posey et al., 2004).

Viable therapy for neuronal dysfunction associated with memory loss (e.g., memory loss that accompanies early stage Alzheimer's disease) is another area with limited success. Long-term potentiation in hippocampal slices however has emerged as a model system for study of physiological and molecular mechanisms that underlie normal learning and memory processes. For example, naturally secreted oligomers of amyloid beta protein (Aβ) have been shown to inhibit long-term potentiation in hippocampal slices.

Huntington's disease (HD) is an autosomal dominant disorder caused by an expanded and unstable CAG trinucleotide repeat that causes a progressive degeneration of neurons, primarily in the putamen, caudate nucleus, and cerebral cortex. HD is characterized by movement abnormalities, cognitive impairments, and emotional disturbances, which eventually culminates in death around 15-20 years after the onset of motor symptoms.

The HD 51 CAG rat utilizes a human HD mutation containing an allele with 51 CAG repeats. This animal model exhibits progressive neurological, neuropathical, and neurochemical phenotypes that closely resemble the late manifesting and slow progression of HD found in humans, and displays HD-like symptoms, such as cognitive impairments and progressive motor dysfunction, as well as neuropathological characteristics, such as neuronal intranuclear inclusions and enlarged lateral ventricles. The HD 51 CAG rat closely resembles the human condition, making it a good candidate to study behavioral, cellular, and molecular markers of the disease.

Known therapies underscore the need for improved approaches to the treatment of autism, Huntington's disease, and associated disorders and/or conditions with compounds that can provide improved efficacy and/or reduced undesirable side effects.

### SUMMARY

A method for treating autism in a patient in need thereof, comprising administering to said patient a pharmaceutically effective amount of a NMDA receptor glycine site partial agonist, is provided herein. For example, the present invention is directed in part to a method for treating autism by administering a therapeutically effective dose of GLYX-13, as disclosed herein, or derivative thereof, for example, a compound having NMDA receptor glycine site partial agonist activity, *e.g.,* a compound that binds to the glycine site of a NMDA receptor, such as a compound disclosed herein. For example, provided herein is a method for treating autism in a patient in need thereof, comprising administering to said patient a pharmaceutically effective amount of a compound that modulates a glycine site on a NMDA receptor.

In other embodiments, methods of treating conditions selected from the group consisting of epilepsy, AIDS, AIDS dementia, multiple system atrophy, progressive supra-nuclear palsy, Friedrich's ataxia, autism (and autism spectrum disorder subtypes), fragile X syndrome, tuberous sclerosis, attention deficit disorder, olivio-ponto-cerebellar atrophy, cerebral palsy, Parkinson's disease, drug-induced optic neuritis, peripheral neuropathy, myelopathy, ischemic retinopathy, glaucoma, cardiac arrest, stroke, ischemia, behavior disorders, impulse control disorders, frontal temporal dementia, schizophrenia and bipolar disorders are provided, comprising administering a NMDA receptor partial agonist such as GLYX-13 or a compound disclosed herein, to a patient in need thereof.

For example, in some embodiments, a contemplated compound that is capable of modulating the glycine site of a NMDA receptor is represented by (GLYX-13): 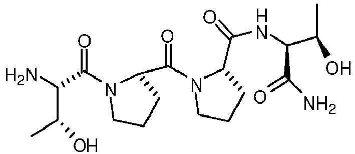 or pharmaceutically acceptable salts thereof.

Also contemplated herein is a method of treating Alzheimer's disease, or a method of treating memory loss that accompanies early stage Alzheimer's disease in a patient in need thereof, comprising administering to the patient a disclosed compound.

In another embodiment, methods are provided herein of treating attention deficit disorder, ADHD, schizophrenia, anxiety, amelioration or treatment of addiction, e.g., of opiate, nicotine and/or ethanol addiction, traumatic brain injury, post-traumatic stress syndrome and/or Huntington's chorea (i.e., Huntington's disease) in a patient in need thereof, comprising administering to the patient a compound disclosed herein.

In some embodiments, a contemplated compound is administered intravenously, intraperitoneally, intramuscularly, or subcutaneously, for example, a contemplated method may include administering a single-dose of said compound. In some embodiments, disclosed methods may provide, after about 1 day, or even after 8 days of administration of a disclosed compound, substantial improvement in one or more autism symptoms of a patient.

In some embodiments, disclosed compounds may be administered daily. A pharmaceutically effective amount of a disclosed compound may be about 0.01mg/kg to about 1000 mg/kg.

Also provided herein is a method of treating autism in a patient in need thereof, comprising administering to said patient a single dose of a compound represented by:

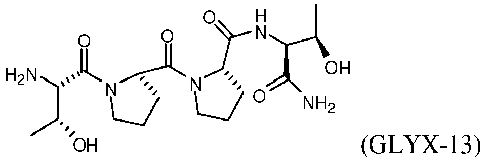

or pharmaceutically acceptable salts thereof, wherein after 1 day, or after 8 days, the patient has substantial improvement in autism symptoms. A single dose may include for example, about 0.01 mg/kg to about 1000 mg/kg of a disclosed compound.

In another embodiment, the disclosed invention relates to administering a dipyrrolidine peptide compound comprising the sequence Thr-Pro-Pro-Thr, or exemplified by Formula A (GLYX-13) for the treatment of autism in mammals including humans.

### BRIEF DESCRIPTION OF THE DRAWINGS

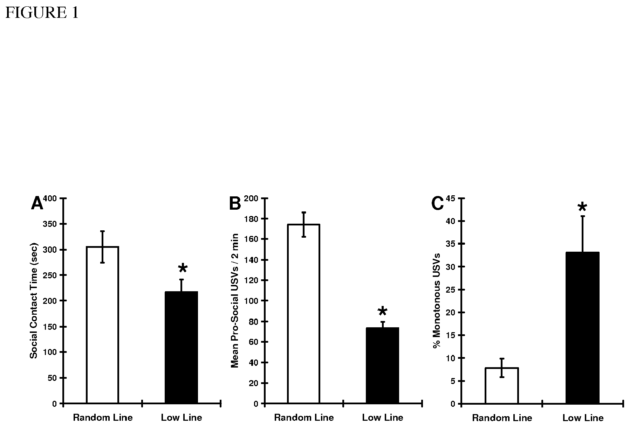

FIGURE 1 depicts Mean ± SEM (A) time spent in direct social contact in adult male Long Evans rats selectively bred for low or random rates of play-induced pro-social USVs. Animals were tested in pairs with a conspecific of the same selectively bred line. (B) Rates of conspecific play induced pro-social USVs (frequency-modulated 50 kHz calls) in adult male low and random-line animals. (C) Proportion of total conspecific play induced USVs that are monotonous (i.e., bandwidth less than 7 kHz; n = 14-20 per group. * P < .05 Fisher PLSD post hoc test, 2 tailed.

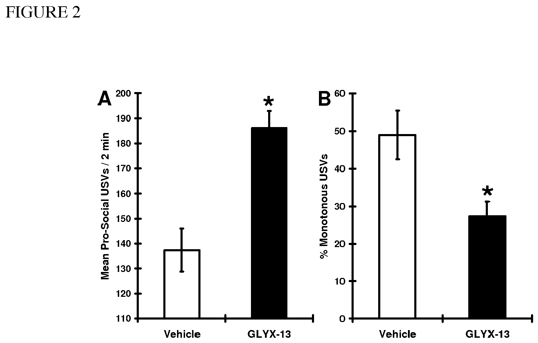

FIGURE 2 depicts Mean ± SEM (A) rates of conspecific play induced pro-social USVs (frequency-modulated 50 kHz calls) in adolescent male low-line rats pretreated with vehicle (1 mg/ml sterile saline s.c.) or (B) GLYX-13 (50 mg/kg s.c.) 15 min before the start of testing using a within-subjects design. (C) Proportion of total conspecific play induced USVs that were monotonous (i.e., bandwidth less than 7 kHz; n = 9 per group. * P < .05 Fisher PLSD post hoc test, 2 tailed.

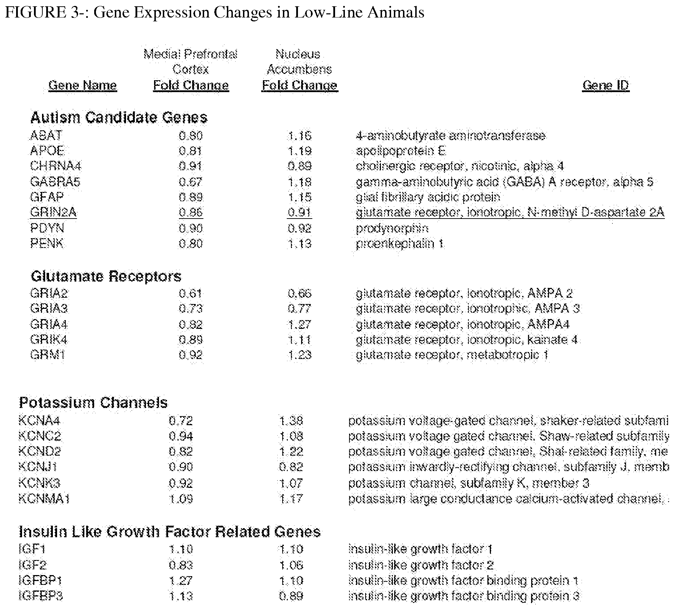

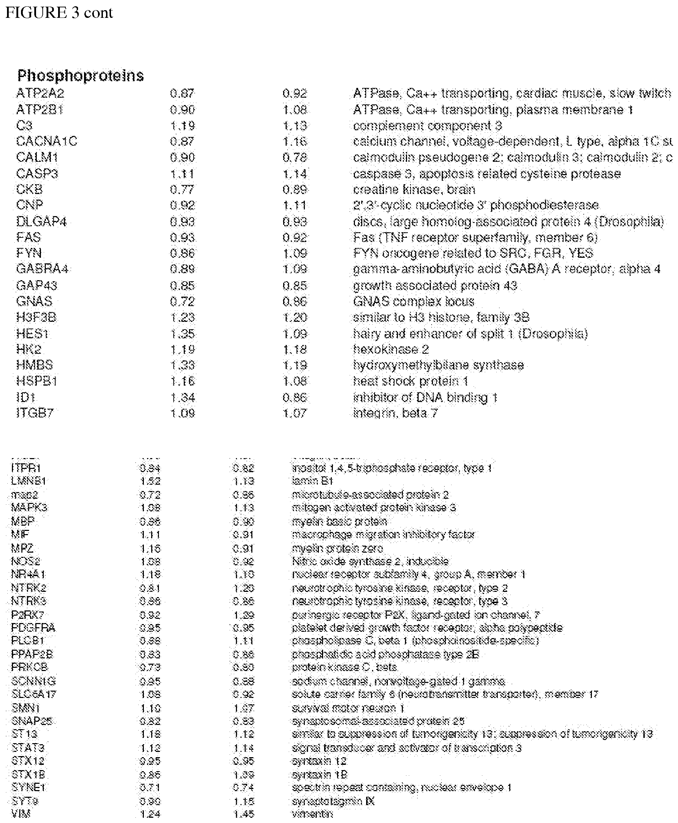

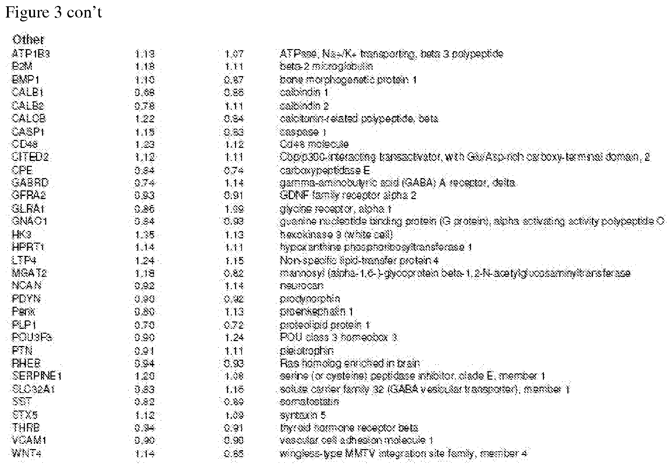

FIGURE 3 depicts significantly changes in genes in two brain regions (medial prefrontal cortex and nucleus accumbens) of low line animals compared to non-selectively bred random-line animals.

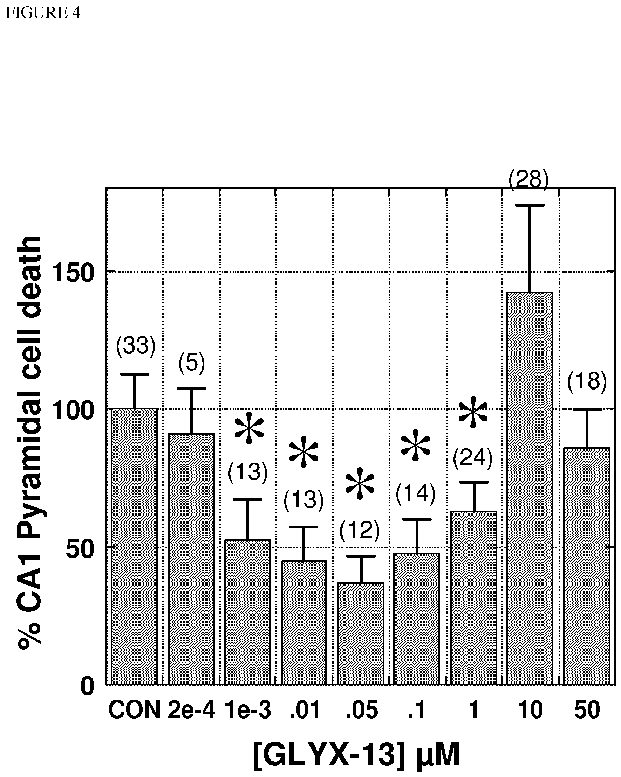

FIGURE 4 depicts delayed neuronal death produced by Aβ(1-42) in organotypic hippocampal slice cultures upon application of GLYX-13; Mean ± SEM propidium fluorescence 24 hr after bath application of Aβ(1-42) alone, versus Aβ(1-42) in the presence of indicated concentrations (µg/ml) of Glyx-13 (*, *P*<.0.05, Student's t-test compared to Aβ alone).

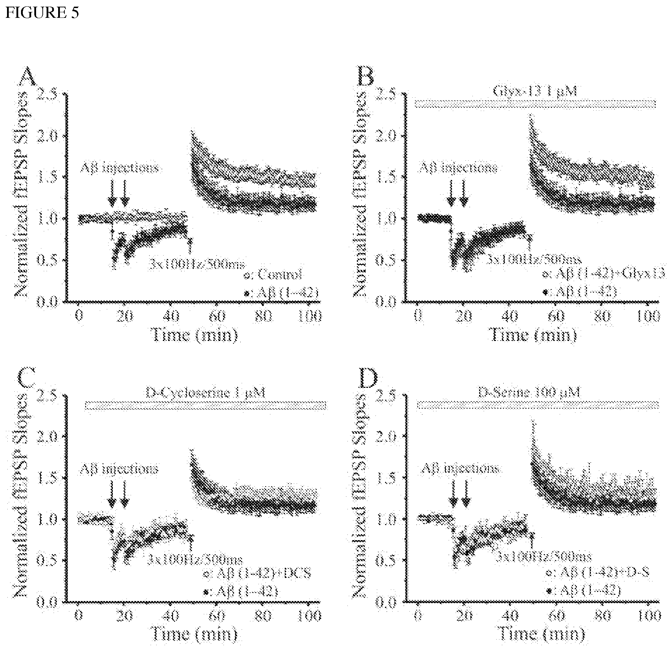

FIGURE 5 depicts rescue of Aβ impairment of long-term potentiation (LTP) of synaptic transmission at Schaffer collateral-CAl synapses in hippocampal slices in presence of GLYX-13. **A:** Time course of the effect of pressure ejection of Aβ(1-42) into stratum radiatum of field CA1 (Aβ injections; 12psi/100ms; filled circles; n=10) on basal synaptic transmission and LTP induced by high-frequency stimulus trains (3x100Hz/500ms), compared to vehicle (TFE) injection (open circles; n=8). **B:** Time course of the effect of pressure ejection of Aβ(1-42) into stratum radiatum of field CA1 in the presence of 1µM Glyx-13 (Aβ injections; 12psi/100ms; filled circles; n=8) on basal synaptic transmission and LTP induced by high-frequency stimulus trains (3x100Hz/500ms), compared to vehicle (TFE) injection (open circles; n=8). **C:** Time course of the effect of pressure ejection of Aβ(1-42) into stratum radiatum of field CA1 in the presence of 1µM D-cycloserine (Aβ injections; 12psi/100ms; filled circles; n=7) on basal synaptic transmission and LTP induced by high-frequency stimulus trains (3x100Hz/500ms), compared to vehicle (TFE) injection (open circles; n=8). **D:** Time course of the effect of pressure ejection of Aβ(1-42) into stratum radiatum of field CA1 in the presence of 100µM D-Serine (Aβ injections; 12psi/100ms; filled circles; n=8) on basal synaptic transmission and LTP induced by high-frequency stimulus trains (3x100Hz/500ms), compared to vehicle (TFE) injection (open circles; n=8). Each point in A, B and C is mean ± SEM normalized fEPSP slope.

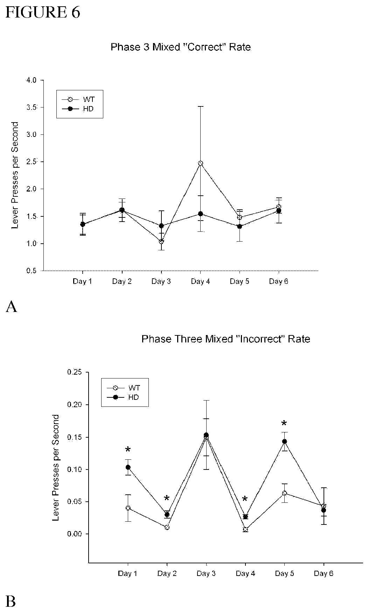

FIGURE 6 indicates the results repeated-measures analysis of variance and a series of one-way ANOVAs between WT and HD rats administered GLYX-13. Figure 6b indicates d no significant differences between WT and HD rats in the "Correct" Rate of lever presses, measured in the amount of lever presses per second, during Phase Three Pseudo-Random testing. Figure 6B shows the, significant difference in the repeated-measure analysis of variance revealed between WT and HD rats in the rate of "Incorrect" lever presses across all testing sessions with the HD rats having a higher "Incorrect" rate (*p* = 0.004).

### DETAILED DESCRIPTION

As used herein, the term "GLYX peptide" refers to a peptide having NMDAR glycine-site partial agonist/antagonist activity. GLYX peptides may be obtained by well-known recombinant or synthetic methods such as those described in US Patents 5,763,393 and 4,086,196 herein incorporated by reference. In some embodiments, GLYX refers to a tetrapeptide having the amino acid sequence Thr-Pro-Pro-Thr (SEQ ID NO: 1), or L-threonyl-L-prolyl-L-prolyl-L-threonine amide.

For example, GLYX-13 refers to the compound depicted as:

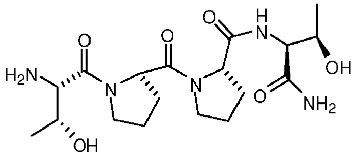

Also contemplated are polymorphs, hydrates, homologs, solvates, free bases, and/or suitable salt forms of GLYX 13 such as, but not limited to, the acetate salt. The peptide may be cyclyzed or non-cyclyzed form as further described in US 5,763,393. In some embodiments, an GLYX-13 analog may include an insertion or deletion of a moiety on one or more of the Thr or Pro groups such as a deletion of CH₂, OH, or NH₂ moiety. In other embodiments, GLYX-13 may be optionally substituted with one or more halogens, C₁-C₃ alkyl (optionally substituted with halogen or amino), hydroxyl, and/or amino. Other compounds contemplated for use herein include Glycine-site partial agonists of the NMDAR disclosed in US 5,763,393, US 6,107,271, and Wood et al., NeuroReport, 19, 1059-1061, 2008, the entire contents of which are herein incorporated by reference.

It may be understood that the peptides disclosed here can include both natural and unnatural amino acids, e.g., all natural amino acids (or derivatives thereof), all unnatural amino acids (or derivatives thereof), or a mixture of natural and unnatural amino acids. For example, one, two, three or more of the amino acids in GLYX-13 may each have, independently, a d- or 1- configuration.

In an embodiment, compounds for use in one or more disclosed methods include those of Formula **I**: 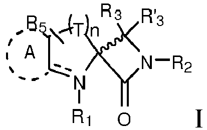 and pharmaceutically acceptable salts, stereoisomers, and N-oxides thereof; wherein
T is, independently for each occurrence, CR₄R₄', and n is 0, 1, 2 or 3;
A is optionally present and is selected from phenyl or pyridine, wherein A is optionally substituted by one or more substituents selected from Rₐ;
R₁ is selected from the group consisting of H, hydroxyl, -S(O)₂-C₁-₄alkyl; -SO₂, C₁-₄alkyl, C₂-C₄alkenyl, phenyl, R₇, or 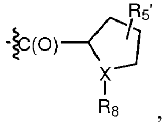 wherein C₁-₄alkyl, C₂-₄alkenyl, or phenyl is optionally substituted by one or more substituents selected from Rₐ;
X is CH or N;
R₃ and R₃ are independently selected from the group consisting of H, halogen, hydroxyl, phenyl, C₁-₄alkyl, amido, amine, or C₂-₄alkenyl, wherein C₁-₄alkyl, C₂-₄alkenyl and phenyl are optionally substituted by one or more substituents selected from Rₐ;
R₄ and R₄ are independently selected from the group consisting of H, halogen, hydroxyl, phenyl, C₁-₄alkyl, amido, amine, C₁-₄alkoxy or C₂-₄alkenyl, wherein C₁-₄alkyl, C₂-₄alkenyl, C₁-₄alkoxy, and phenyl are optionally substituted by one or more substituents selected from Rₐ;
R₂ is selected from the group consisting of H, R₇, -S(O)₂, S(O)₂-C₁-C₄alkyl, C₁-₄alkyl, hydroxyl, or phenyl wherein C₁-₄alkyl, C₂₋₄alkenyl and phenyl are optionally substituted by one or more substituents selected from Rₐ;
R₅ and R₅' are each independently selected from group consisting of H, halogen, C₁-₄alkyl, C₁-₄alkoxy, C₂-₄alkenyl, cyano, amino, phenyl, and hydroxyl, wherein C₁-₄alkyl, C₂-₄alkenyl and phenyl are optionally substituted by one or more substituents selected from Rₐ;
R₇ is selected from group consisting of -C(O)-R₉, -C(O)-O-R₉, or -C(O)-NR_{d}-R₉,
R₉ is selected from the group consisting of H, C₁-₄alkyl, phenyl, or heterocyclic, wherein C₁-₄alkyl, phenyl or heterocyclic is optionally substituted by 1, 2 or 3 substituents selected from R_{b}
R₈ is selected from group consisting of H, -C(O)- C₁-₄alkyl or C(O)-O- C₁-₄ alkyl, wherein C₁-₄alkyl is optionally substituted by 1, 2 or 3 substituents selected from Rₐ;
Rₐ is selected, independently for each occurrence, from carboxy, hydroxyl, halogen, amino, phenyl, C₁-₄alkyl, and C₁-₄alkoxy;
R_{b} is selected, independently for each occurrence, from the group consisting of carboxy, hydroxyl, halogen, amino, phenyl, C₁-₄alkyl, C₁-₄alkoxy, and -NH-R_{c};
R_{c} is selected, independently for each occurrence from the group consisting of: -C(O)-O-C₁-₄alkyl; and -C(O)-C₁-₄alkyl; and
R_{d} is selected, independently for each occurrence, H and C₁-₄alkyl;
and pharmaceutically acceptable salts, N-oxides or stereoisomers thereof.

For example, contemplated compounds include:

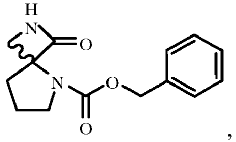

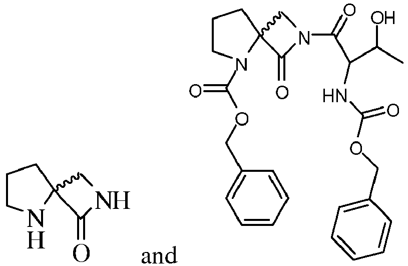

Disclosed herein are compounds selected from the group consisting of: 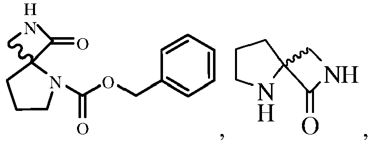 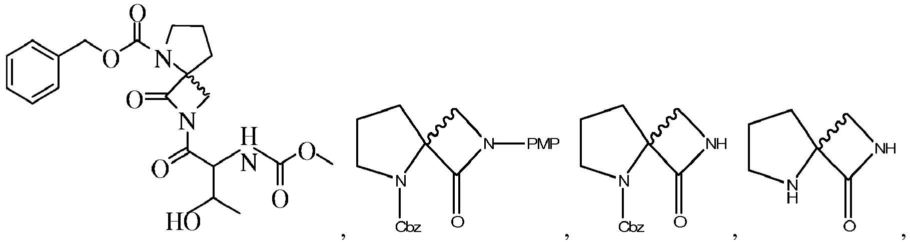 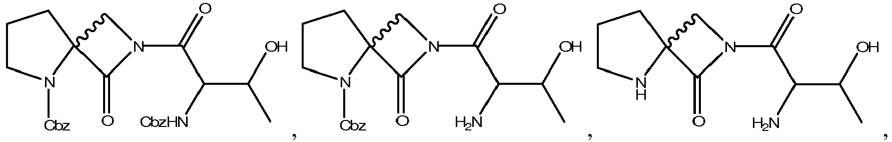 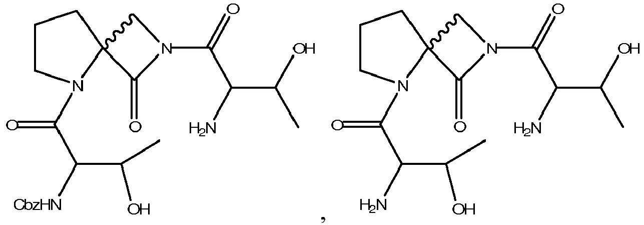 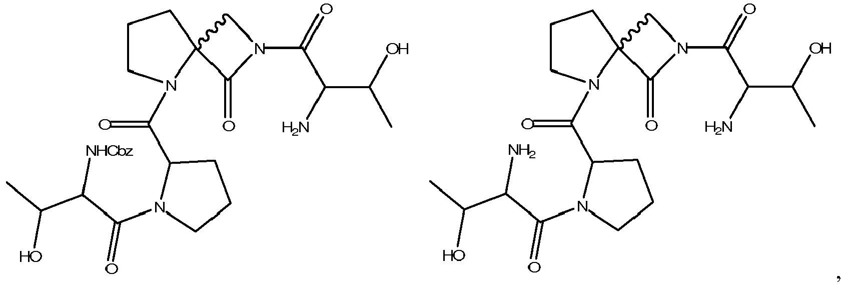 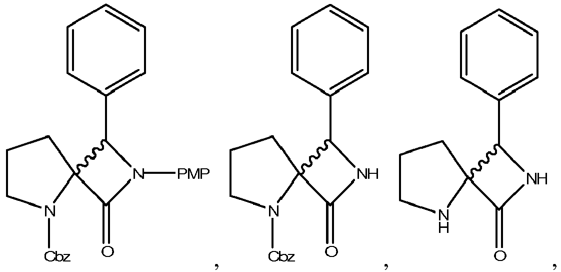 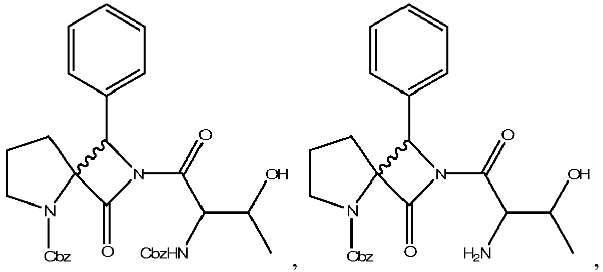 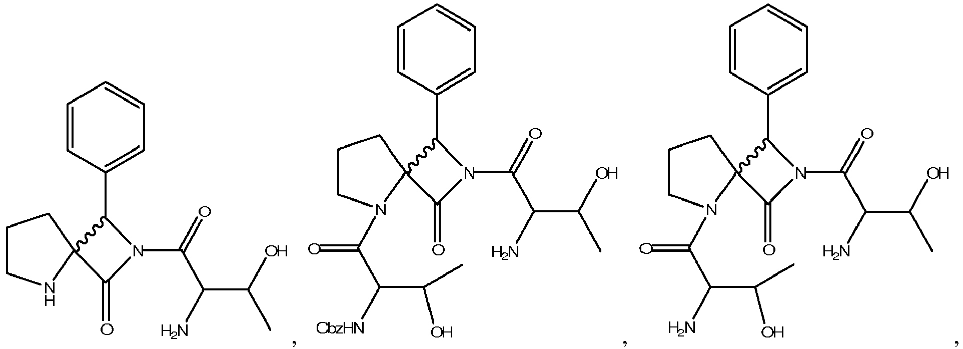 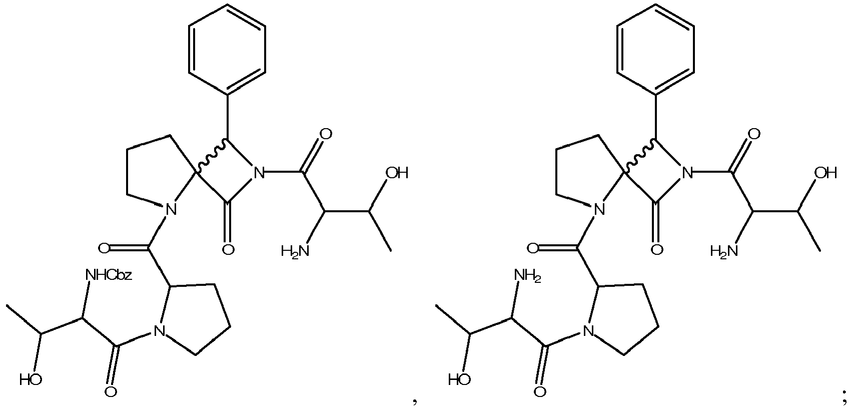 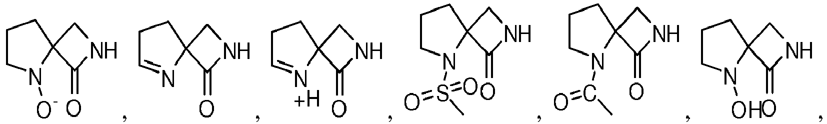 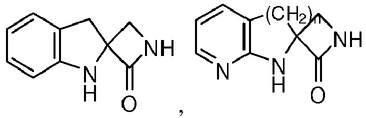 (where n is 0, 1, 2 or 3), and

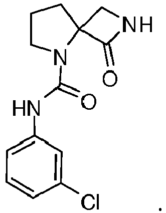

### Methods

Contemplated methods include a method of treating autism and/or an autism spectrum disorder in a patient need thereof, comprising administering an effective amount of a disclosed compound (e.g. a compound described above) to the patient. In an embodiment, a method for reducing the symptoms of autism in a patient in need thereof is contemplated, comprising administering an effective amount of a disclosed compound to the patient. For example, upon administration, the compound may decrease the incidence of one or more symptoms of autism such as eye contact avoidance, failure to socialize, attention deficit, poor mood, hyperactivity, abnormal sound sensitivity, inappropriate speech, disrupted sleep, and perseveration. Such decreased incidence may be measured relative to the incidence in the untreated individual or an untreated individual(s).

In some embodiments, patients suffering from autism also suffer from another medical condition, such as Fragile X syndrome, tuberous sclerosis, congenital rubella syndrome, and untreated phenylketonuria.

In another embodiment, methods of treating a disorder in a patient need thereof are contemplated, wherein the disorder is selected from group consisting of: epilepsy, AIDS and/or AIDS dementia, Parkinson's disease, multiple system atrophy, progressive supra-nuclear palsy, Friedrich's ataxia, autism, fragile X syndrome, tuberous sclerosis, attention deficit disorder, olivio-ponto-cerebellar atrophy, cerebral palsy, drug-induced optic neuritis, peripheral neuropathy, myelopathy, ischemic retinopathy, glaucoma, cardiac arrest, behavior disorders, and impulse control disorders that includes administering a disclosed compound, e.g GLYX-13. Also contemplated herein is a method for treating cough, e.g. uncontrollable cough, comprising administering GLYX-13 to a patient in need thereof.

For example, provided here are methods of treating benign Rolanic epilepsy, frontal lobe epilepsy, infantile spasms, juveline myoclonic epilepsy, Lennox-Gastaut syndrome, Landau-Kleffner syndrome, Dravet syndrome, progressive myoclonus epilepsies, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Lafora disease, and /or photosensitive epilepsy comprising administering an effective amount of a disclosed compound.

In an embodiment, contemplated herein are methods of treating attention deficit disorder, ADHD (attention deficit hyperactivity disorder), schizophrenia (for example, schizo-affective disorders, delusional disorders, e.g., paranoid type, hebephrenic, and/or catatonic type schizophrenia), bipolar disorder (include bipolar **I** disorder, bipolar **II** disorder, cyclothymia), borderline personality disorder, anxiety,(include social anxiety disorder, avoidant personality disorder), obsessive-compulsive disorder, amelioration of opiate, nicotine and/or ethanol addiction (e.g., method of treating such addiction or ameliorating the side effects of withdrawing from such addiction), spinal cord injury diabetic retinopathy, traumatic brain injury, frontal temporal dementia, post-traumatic stress syndrome and/or Huntington's chorea, in a patient in need thereof, that includes administering a disclosed compound, e.g., GLYX-13. For example, patients suffering from schizophrenia, addiction (e.g. ethanol or opiate), autism (and autism spectrum disorders), Huntington's chorea, traumatic brain injury, spinal cord injury, post-traumatic stress syndrome and diabetic retinopathy may all be suffering from altered NMDA receptor expression or functions.

For example, provided herein is a method of treating schizophrenia, for example, the negative and cognitive symptoms of schizophrenia in a patient suffering therefrom, comprising administering a therapeutically effective amount of a disclosed compound.

Contemplated here is a method of treating stroke and/or ischemia, e.g., ischemic stroke, brain ischemia, transient ischemic attack, cardiac ischemia,and/or myocardial infarction, in a patient in need thereof, comprising administering a pharmaceutically effective amount of GLYX-13.

Also provided herein is a method of modulating an autism target gene expression in a cell comprising contacting a cell with an effective amount of a disclosed compound, e.g GLYX-13. The autism gene expression may be for example, selected from ABAT, APOE, CHRNA4, GABRA5,GFAP, GRIN2A, PDYN, and PENK. In another embodiment, a method of modulating synaptic plasticity in a patient suffering from a synaptic plasticity related disorder is provided, comprising administering to the patient an effective amount of a disclosed compound, such as GLYX-13, or pharmaceutically acceptable salts thereof.

In another embodiment, a method of treating Alzheimer's disease, or e.g., treatment of memory loss that e.g., accompanies early stage Alzheimer's disease, in a patient in need thereof is provided, comprising administering a disclosed compound, e.g., GLYX-13. Also provided herein is a method of modulating an Alzheimer's amyloid protein (e.g., beta amyloid peptide, e.g. the isoform Aβ₁₋₄₂), in-vitro or in-vivo (e.g. in a cell) comprising contacting the protein with an effective amount of a disclosed compound, e.g GLYX-13. For example, in some embodiments, GLYX-13 or another disclosed compound may block the ability of such amyloid protein to inhibit long-term potentiation in hippocampal slices as well as apoptotic neuronal cell death. In some embodiments, a disclosed compound (e.g., GLYX-13) may provide neuroprotective properties to a Alzheimer's patient in need thereof, for example, may provide a therapeutic effect on later stage Alzheimer's -associated neuronal cell death.

In some embodiments, the patient is a human, e.g. a human pediatric patient.

In some embodiments, contemplated methods relate to use of a disclosed compound or compounds alone or in combination with one or more other agents for manufacturing a medicament for treating autism or another contemplated indication. In an embodiment, the disclosure relates to methods for treating autism by administering an effective amount of GLYX-13 to a patient in need thereof.

For example, in a disclosed method, a contemplated compound, *e.g.,* GLYX-13, or a composition comprising a contemplated compound and, *e.g.,* a pharmaceutically acceptable excipient, may be administered parenterally to a patient including, but not limited to, subcutaneously and intravenously. The compound or compositions of the invention may also be administered via slow controlled i.v. infusion or by release from an implant device. In an embodiment, a disclosed method for treating autism includes administering one dose, or one or more doses, of a disclosed compound. In some embodiments, a patient has substantial improvement in autism symptoms after 12 hours, after 1 day, after 1 week, after 2 days, after 3 days, after 4 days, after 5 days, after 6 days, or even after 8 days of a one (single) dose administration.

A therapeutically effective amount of a disclosed compound required for use in therapy varies with the nature of the autism condition being treated, the length of treatment time desired, the age and the condition of the patient, and is ultimately determined by the attending physician. In general, however, doses employed for adult human treatment typically are in the range of about 0.01 mg/kg to about 1000 mg/kg per day. The dose may be about 1 mg/kg to about 100 mg/kg per day. The desired dose may be conveniently administered in a single dose, or as multiple doses administered at appropriate intervals, for example as two, three, four or more sub-doses per day.

A number of factors may lead to the compounds of a disclosed invention being administered over a wide range of dosages. When given in combination with other therapeutic agents, the dosage of the compounds of the present invention may be given at relatively lower dosages. As a result, the dosage of a compound of the present invention may be from about 1 ng/kg to about 100 mg/kg. The dosage of a compound of the present invention may be at any dosage including, but not limited to, about 1 ug/kg, 25 ug/kg, 50 ug/kg, 75 ug/kg, 100 u ug/kg, 125 ug/kg, 150 ug/kg, 175 ug/kg, 200 ug/kg, 225 ug/kg, 250 ug/kg, 275 ug/kg, 300 ug/kg, 325 ug/kg, 350 ug/kg, 375 ug/kg, 400 ug/kg, 425 ug/kg, 450 ug/kg, 475 ug/kg, 500 ug/kg, 525 ug/kg, 550 ug/kg, 575 ug/kg, 600 ug/kg, 625 ug/kg, 650 ug/kg, 675 ug/kg, 700 ug/kg, 725 ug/kg, 750 ug/kg, 775 ug/kg, 800 ug/kg, 825 ug/kg, 850 ug/kg, 875 ug/kg, 900 ug/kg, 925 ug/kg, 950 ug/kg, 975 ug/kg, 1 mg/kg, 5 mg/kg, 10 mg/kg, 15 mg/kg, 20 mg/kg, 25 mg/kg, 30 mg/kg, 35 mg/kg, 40 mg/kg, 45 mg/kg, 50 mg/kg, 60 mg/kg, 70 mg/kg, 80 mg/kg, 90 mg/kg, or 100 mg/kg.

In some embodiments, the disclosed compound, e.g. GLYX-13, may be dosed at amount that produces antidepressive-like and/or anxiolytic-like effects.

Disclosed compounds may be provided as part of a liquid or solid formulation, for example, aqueous or oily suspensions, solutions, emulsions, syrups, and/or elixirs. The compositions may also be formulated as a dry product for constitution with water or other suitable vehicle before use. Such liquid preparations may contain additives including, but not limited to, suspending agents, emulsifying agents, nonaqueous vehicles and preservatives. Suspending agent include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl hydroxybenzoate and sorbic acid. Contemplated compounds may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

### EXAMPLES

### Example 1: USVs of Low-Line versus Random-Line Rats

Core symptoms of autism (1) deficits in social emotions and motivations, 2) communication problems, and 3) a high incidence of motor abnormalities, especially repetitive behaviors) can be modeled in part in animals using the following behavioral measures, respectively: (1) reduced time spent in social contact; (2) reduced rates of pro-social vocalizations, and (3) disrupted ultrasonic vocalization patterns.

Adult male Long Evans rats were selectively bred for low or random rates of play-induced pro-social ultrasonic vocalizations (USVs) (i.e. frequency modulated 50-kHZ USV in response to rough and tumble play behaviors). Animals were tested in pairs with a conspecific of the same selectively bred line. FIGURE 1A depicts the Mean ± SEM time these rats spent in direct social contact. FIGURE 1B illustrates the rates of conspecific play induced pro-social USVs (frequency-modulated 50 kHz calls) in adult male low and random-line animals. FIGURE 1C illustrates the proportion of total conspecific play induced USVs that are monotonous (i.e., bandwidth less than 7 kHz). Frequency modulated 50-kHz calls have been shown to be associated with pro-social positive affective states; monotonous calls have been associated with communication deficits). Data adapted and reanalyzed from Burgdorf et al. Dev. Psychobiol 51, 34-46 (2009). n = 14-20 per group. * P < .05 Fisher PLSD post hoc test, 2 tailed.

The low-line animals engaged in less social contact time with conspecifics, showed lower rates of play induced pro-social USVs, and exhibited an increased proportion of monotonous USVs compared to randomly bred animals when tested as adults. e,g., this example illustrates that this animal model displays the core social and communicative symptoms of autism.

### Example 2: Effect of GLYX-13 on Conspecific Play Induced Pro-social USVs

As shown in FIGURE 2A, conspecific play induced pro-social USVs (frequency-modulated 50 kHz calls) were measured in adolescent male low-line rats pretreated with vehicle (1 mg/ml sterile saline s.c.). FIGURE 2B shows the results of treatment with GLYX-13 (50 mg/kg s.c.) 15 min before the start of testing using a within-subjects design. The proportion of total conspecific play induced USVs that were monotonous (i.e., bandwidth less than 7 kHz) is shown in FIGURE 2C.. n = 9 per group. * P < .05 Fisher PLSD post hoc test, 2 tailed. GLYX-13 significantly increased rates of play inducing pro-social USVs and significantly decreased the proportion of total USVs that are monotonous.

These results indicate that GLYX-13 can reverse the social / emotional communication deficit in low-line rats.

### Example 3: Gene Expression Changes in Low-Line Animals

Gene expression changes in low-line animals were examined by microarray using methods as previously described in Burgdorf et al., Neuroscience 168, 769-777 (2010) in two brain regions (medial prefrontal cortex and nucleus accumbens) that have been shown to be functionally linked to play induced pro-social vocalizations (Burgdorf et al., Behav Brain Res 182, 274-283 (2007).

The list of significantly changed genes in both brain regions in adult low-line animals compared to non-selectively bred adult random-line animals appears in Figure 3. There is statistically significant overlap between the human autism associated genes and genes differentially expressed in low-line animals (8 changed autism candidate genes / 101 total changed genes representing a 3.5 fold enrichment compared to the chip set bias of 25 / 1108; P < . 05; Chi square test, two tailed).

The NMDA receptor was identified as a hub in the genes differentially expressed in the low-line animals [Mean ± SEM (0.985 ± 0.004) average connections per gene differentially expressed in low-line animals compared to 3 connections for the NR2A subunit of the NMDA receptor; P < .0001 one tailed t-test]. As a negative control, the cortical microarray data from rats that received social defeat which is not thought to model autism was also analyzed, and found that neither the autism candidate genes were upregulated in this gene set, nor was the NMDAR identified as a hub gene (all P's > .05).

Gene expression profiling was conducted by microarray as described in Burgdorf et al. Neuroscience (2010) in the accumbens and medial prefrontal cortex of adult male Long Evans rats selectively bred for low rates of 50-kHz USVs and non-selectively bred random-line controls. n = 6 per group. The false discovery rate was set at 5%. Low-line animals showed a significant enrichment in human autism associated genes (2.9 fold enrichment vs. chip set bias, P < .05.

### Example 4 Protection Against human b-amyloid(1-42) toxicity

To determine whether Glyx-13, by renormalizing activation of NMDA receptors, might protect against the toxicity of hAβ to susceptible neurons in a physiological network milieu, we compared the long-term effects of hAβ in the absence and presence of different concentrations of Glyx-13. As shown in Figure 4, a 5µg/ml concentration of hAβ alone produced marked delayed death of glutamatergic pyramidal neurons in field CA1 24 hr after extracellular application. Application of a mixture of the same concentration of hAβ and Glyx-13 demonstrated that Glyx-13 exhibits a U-shaped dose-response relation of neuroprotective potency, with concentrations as low as 1 nM up to 1µM Glyx-13 showing significant protection against hAβ-induced death of CA1 pyramidal neurons (*, *P*<0.05, Duncan multiple range t-test compared to hAβ alone.

### Example 5 hAb-induced impairements in long term potentiation (LTP) at Schaffer collateral-CAl synapses

Since LTP is believed to be an important mechanism of long-term memory storage, acute synaptotoxic action of hAβ could have tremendous import for the cognitive decline and memory impairments that are a hallmark of *early* stage AD. Therefore, an important question is whether haBC also potentiates this functional synaptotoxic action of hAβ, and whether a glycine site partial agonist can protect synapses from this toxicity.

We pressure ejected hAβ in the absence or presence of Glyx-13, D-cycloserine or the full agonist D-serine, into the Schaffer collateral synaptic field in CA1 *stratum radiatum* of acute *in vitro* rat hippocampal slices and attempted to evoke LTP 30 min later. Figure 5A illustrates that hAβ alone caused a significant reduction (∼20%) in the magnitude of LTP elicited by high-frequency Schaffer collateral stimulation. When hAβ was pressure ejected in the presence of bath-applied Glyx-13 (1µM, Fig. 5B, open circles), LTP was restored to normal control levels. In contrast, neither the glycine site partial agonist D-cycloserine (1µM, Fig. 5C, open circles) nor the full agonist D-serine (100µM, Fig. 5D, open circles) was able to rescue LTP compared to synapses treated with hAβ injections alone (filled circles). These data show that Glyx-13 exhibits a potent ability to protect LTP at synapses from the acute synaptotoxic effects of hAβ, suggesting that Glyx-13 may be both an effective nootropic agent in early stage Alzheimer's disease, and a neuroprotective agent later in the disease process.

### Example 6- Huntington's disease model

HD 51 CAG and WT (wild type) rats are used in a Huntington's disease model study, testing cognition with serial operant reversal tasks. Rats are dosed according to the following schedule:

| - | **GLYX-13 Dosage Schedule** | | | |
|---|---|---|---|---|
| **Genotype** | **Saline** | **1mg/kg** | **5mg/kg** | **10mg/kg** |
| Homozygote "HD" | n=7 | n=0 | n=9 | n=3 |
| Wild-type "WT" | n=7 | n=0 | n=6 | n=0 |

The animals are first placed in an acquisition trial. The acquisition trial is an 8-hour testing period where the animal is reinforced with a sucrose food pellet for a "correct" lever press after a variable interval time of 30 seconds. This task requires the animals to learn which lever needs to be activated to gain reinforcement. The time taken to identify the "correct" lever is measured, as well as the number of reinforcers earned, percentage of "correct" lever presses, and errors made (incorrect lever presses). The acquisition trial is performed with access to water *ad libidum.*

Following the acquisition trial, the animals are trained on a progressive fixed ratio (FR) schedule of reinforcement, using increments of one lever presses until they are pressing the lever 15 times to receive a reinforce (i.e., an FR15), which typically takes three days. For all training and testing sessions, the operant program will be running for a maximum of 60 minutes or until the rat achieves a maximum of 200 sucrose pellets, upon which time they will be immediately removed from the chambers and placed back into their home cage. The MED PC IV program is used to increase the FR in small increments depending on the performance of the rat (i.e. the rat will have the FR increased to FR2 after achieving 15 reinforcers on FR1. Following successful progressive FR training, the rats are put into the chambers for two consecutive days of training on FR16 help establish a baseline response rate and to allow the animal to become accustomed to multiple lever presses to gain reinforcement.

The testing session is conducted using a progressive FR schedule for 25 or 30 days. The first phase of testing consists of nine days of FR, with the left lever being the "correct" lever for reinforcement. The rats start on a FR2 and then move to the next increased FR each day, until they reach FR512 (2, 4, 8, 16, 32, 64, 128, 256, and 512, respectively). The response rate for the left and right lever is recorded, as well as the total number of lever presses for each side, the total number of lever presses combined for each side in a session, the percentage of "correct" lever presses ("correct" lever presses/left lever presses + right lever presses then multiplied by 100 to obtain a percentage), the total reinforcers earned, and the time to complete each session. The rats are given one day off before the second phase of testing begins, with only the weight of the rats being recorded. During phase **I,** both the HD and WT animals press the correct lever in proportion to total presses.

The second phase of testing consists of nine days of FR testing, with the right lever now becoming the "correct" lever for reinforcement. The second phase of testing is identical to the first phase, with the exception of the spatial reversal of the "correct" lever. The same measures are recorded. During phase **II,** the correct response lever is switched to the right side. Both HD and WT animals press the correct lever similarly in proportion to total presses. However, the HD animals show preservative errors with increased incorrect lever presses on day 2.

Upon completion of the second phase of the testing, the rats are given a day off, with only their weights being recorded and enter a pseudo-random mixed schedule. The pseudo-random phase consists of rats performing a FR32 (when the animals are responding at the highest response rate) for six days with the "correct" being switched pseudo-randomly (e.g., Left, Left, Right, Right, Left, and Right). During phase 3, GLYX-13 was delivered subcutaneously 20 minutes prior to start of task, using dosage schedule above. Both WT and HD animals administered GLYX-13 show higher percentage trends of correct lever presses than animals with saline injections. See Figure 6.

Figure 6A indicates repeated-measures analysis of variance and a series of one-way ANOVAs revealed no significant differences between WT and HD rats in the "Correct" Rate of lever presses, measured in the amount of lever presses per second, during Phase Three Pseudo-Random testing. However, the repeated-measure analysis of variance revealed a significant difference between WT and HD rats in the rate of "Incorrect" lever presses across all testing sessions with the HD rats having a higher "Incorrect" rate (*p* = 0.004). (Figure 6B) The one-way ANOVA revealed significant differences between HD and WT rats when measured for "Incorrect" rate during day 1 (*p* = 0.05), day 2 (*p* = 0.026), day 4 (*p* = 0.013), and day 5 (*p* = 0.018) with the HD rats having a higher rate for each day.

At the conclusion of operant testing, the rats are placed into the open field motor monitor system (Kinder Scientific; Poway, CA). The rats are then tested during their nocturnal phase for one hour, and the total distance traveled, basic and fine movements performed, the proximity to the center, and the number of rears (i.e., standing on hind legs) is recorded. Total distance traveled indicates if the HD 51 CAG rats exhibit any hyper- or hypoactivity, relative to the wild-type rats. Total number of rearing events is an additional measure of exploratory behavior and activity levels, but can also help detect motor abnormalities.

### EQUIVALENTS

Those skilled in the art will recognize, or be able to ascertain using no more than routine experimentation, many equivalents to the specific embodiments of the invention described herein. Such equivalents are intended to be encompassed by the following claims.

### INCORPORATION BY REFERENCE

The entire contents of all patents, published patent applications, websites, and other references cited herein are hereby expressly incorporated herein in their entireties by reference.

## Claims

1. A method for treating autism in a patient in need thereof, comprising administering to said patient:
a pharmaceutically effective amount of GLYX-13: 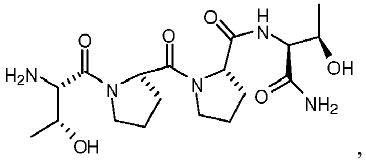 or pharmaceutically acceptable salt thereof, or
a pharmaceutically effective amount of a compound represented by Formula **I**: 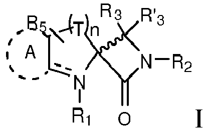 and pharmaceutically acceptable salts, stereoisomers, and N-oxides thereof; wherein
T is, independently for each occurrence, CR₄R₄', and n is 0, 1, 2 or 3;
A is optionally present and is selected from phenyl or pyridine, wherein A is optionally substituted by one or more substituents selected from Rₐ;
R₁ is selected from the group consisting of H, hydroxyl, -S(O)₂-C₁-₄alkyl; -SO₂, C₁-₄alkyl, C₂-C₄alkenyl, phenyl, R₇, or 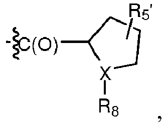 wherein C₁-₄alkyl, C₂-₄alkenyl, or phenyl is optionally substituted by one or more substituents selected from Rₐ;
X is CH or N;
R₃ and R₃ are independently selected from the group consisting of H, halogen, hydroxyl, phenyl, C₁-₄alkyl, amido, amine, or C₂-₄alkenyl, wherein C₁-₄alkyl, C₂-₄alkenyl and phenyl are optionally substituted by one or more substituents selected from Rₐ;
R₄ and R₄ are independently selected from the group consisting of H, halogen, hydroxyl, phenyl, C₁-₄alkyl, amido, amine, C₁-₄alkoxy or C₂-₄alkenyl, wherein C₁-₄alkyl, C₂-₄alkenyl, C₁-₄alkoxy, and phenyl are optionally substituted by one or more substituents selected from Rₐ;
R₂ is selected from the group consisting of H, R₇, -S(O)₂, S(O)₂-C₁-C₄alkyl, C₁-₄alkyl, hydroxyl, or phenyl wherein C₁-₄alkyl, C₂₋₄alkenyl and phenyl are optionally substituted by one or more substituents selected from Rₐ;
R₅ and R₅' are each independently selected from group consisting of H, halogen, C₁-₄alkyl, C₁-₄alkoxy, C₂-₄alkenyl, cyano, amino, phenyl, and hydroxyl, wherein C₁-₄alkyl, C₂-₄alkenyl and phenyl are optionally substituted by one or more substituents selected from Rₐ;
R₇ is selected from group consisting of -C(O)-R₉, -C(O)-O-R₉, or -C(O)-NR_{d}-R₉,
R₉ is selected from the group consisting of H, C₁-₄alkyl, phenyl, or heterocyclic, wherein C₁-₄alkyl, phenyl or heterocyclic is optionally substituted by 1, 2 or 3 substituents selected from R_{b}
R₈ is selected from group consisting of H, -C(O)- C₁-₄alkyl or C(O)-O-C₁-₄ alkyl, wherein C₁-₄alkyl is optionally substituted by 1, 2 or 3 substituents selected from Rₐ;
Rₐ is selected, independently for each occurrence, from carboxy, hydroxyl, halogen, amino, phenyl, C₁-₄alkyl, and C₁-₄alkoxy;
R_{b} is selected, independently for each occurrence, from the group consisting of carboxy, hydroxyl, halogen, amino, phenyl, C₁-₄alkyl, C₁-₄alkoxy, and -NH-R_{c};
R_{c} is selected, independently for each occurrence from the group consisting of: -C(O)-O-C₁-₄alkyl; and -C(O)-C₁-₄alkyl; and
R_{d} is selected, independently for each occurrence, H and C₁-₄alkyl;
and pharmaceutically acceptable salts, N-oxides or stereoisomers thereof.

2. The method of claim 1, wherein about 1 day after administration, the patient has substantial improvement in one or more autism symptoms.

3. The method of any one of claims 1-2, wherein about 8 days after administration the patient has substantial improvement in one or more autism symptoms

4. A method of treating a condition selected from the group consisting of epilepsy, AIDS dementia, multiple system atrophy, progressive supra-nuclear palsy, Friedrich's ataxia, Down's syndrome, fragile X syndrome, tuberous sclerosis, olivio-ponto-cerebellar atrophy, cerebral palsy, drug-induced optic neuritis, peripheral neuropathy, myelopathy, ischemic retinopathy, diabetic retinopathy, glaucoma, cardiac arrest, behavior disorders, and impulse control disorders, in a patient in need thereof, comprising administering to said patient:
a pharmaceutically effective amount of GLYX-13: 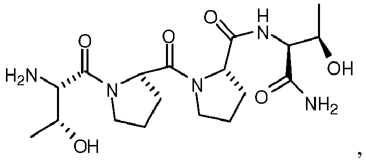 or pharmaceutically acceptable salt thereof, or
a pharmaceutically effective amount of a compound represented by Formula **I**: 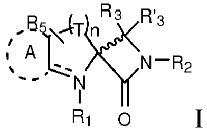 wherein
T is, independently for each occurrence, CR₄R₄', and n is 0, 1, 2 or 3;
A is optionally present and is selected from phenyl or pyridine, wherein A is optionally substituted by one or more substituents selected from Rₐ;
R₁ is selected from the group consisting of H, hydroxyl, -S(O)₂-C₁-₄alkyl; -SO₂, C₁-₄alkyl, C₂-C₄alkenyl, phenyl, R₇, or 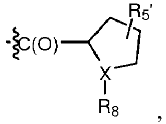 wherein C₁-₄alkyl, C₂-₄alkenyl, or phenyl is optionally substituted by one or more substituents selected from Rₐ;
X is CH or N;
R₃ and R₃ are independently selected from the group consisting of H, halogen, hydroxyl, phenyl, C₁-₄alkyl, amido, amine, or C₂-₄alkenyl, wherein C₁-₄alkyl, C₂-₄alkenyl and phenyl are optionally substituted by one or more substituents selected from Rₐ;
R₄ and R₄ are independently selected from the group consisting of H, halogen, hydroxyl, phenyl, C₁-₄alkyl, amido, amine, C₁-₄alkoxy or C₂-₄alkenyl, wherein C₁-₄alkyl, C₂-₄alkenyl, C₁-₄alkoxy, and phenyl are optionally substituted by one or more substituents selected from Rₐ;
R₂ is selected from the group consisting of H, R₇, -S(O)₂, S(O)₂-C₁-C₄alkyl, C₁-₄alkyl, hydroxyl, or phenyl wherein C₁-₄alkyl, C₂₋₄alkenyl and phenyl are optionally substituted by one or more substituents selected from Rₐ;
R₅ and R₅' are each independently selected from group consisting of H, halogen, C₁-₄alkyl, C₁-₄alkoxy, C₂-₄alkenyl, cyano, amino, phenyl, and hydroxyl, wherein C₁-₄alkyl, C₂-₄alkenyl and phenyl are optionally substituted by one or more substituents selected from Rₐ;
R₇ is selected from group consisting of -C(O)-R₉, -C(O)-O-R₉, or -C(O)-NR_{d}-R₉,
R₉ is selected from the group consisting of H, C₁-₄alkyl, phenyl, or heterocyclic, wherein C₁-₄alkyl, phenyl or heterocyclic is optionally substituted by 1, 2 or 3 substituents selected from R_{b}
R₈ is selected from group consisting of H, -C(O)- C₁-₄alkyl or C(O)-O-C₁-₄ alkyl, wherein C₁-₄alkyl is optionally substituted by 1, 2 or 3 substituents selected from Rₐ;
Rₐ is selected, independently for each occurrence, from carboxy, hydroxyl, halogen, amino, phenyl, C₁-₄alkyl, and C₁-₄alkoxy;
R_{b} is selected, independently for each occurrence, from the group consisting of carboxy, hydroxyl, halogen, amino, phenyl, C₁-₄alkyl, C₁-₄alkoxy, and -NH-R_{c};
R_{c} is selected, independently for each occurrence from the group consisting of: -C(O)-O-C₁-₄alkyl; and -C(O)-C₁-₄alkyl; and
R_{d} is selected, independently for each occurrence, H and C₁-₄alkyl;
and pharmaceutically acceptable salts, N-oxides or stereoisomers thereof.

5. The method of claim 4, wherein the condition is fragile X syndrome.

6. The method of any one of claims 1-5, wherein the compound is administered intravenously, intraperitoneally, intranasally, orally, intramuscularly, or subcutaneously.

7. The method of any one of claims 1-6, wherein the method comprises administering a single-dose of said compound.

8. A method of modulating an autism target gene expression in a cell comprising contacting a call with an effective amount of the compound: 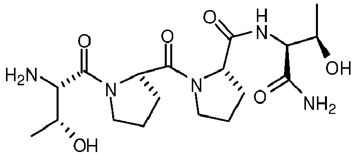 or pharmaceutically acceptable salts thereof.

9. The method of claim 8, wherein the autism target is selected from ABAT, APOE, CHRNA4, GABRA5,GFAP, GRIN2A, PDYN, an d PENK.

10. A method of modulating synaptic plasticity in a patient suffering from a synaptic plasticity related disorder, comprising administering to the patient an effective amount of the compound: 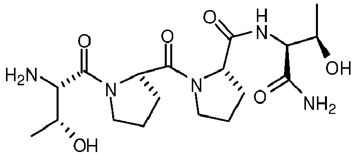 or pharmaceutically acceptable salts thereof.

11. The method of any one of claims 1-10, wherein the patient is a pediatric patient.

12. A method of treating attention deficit disorder, ADHD, schizophrenia, anxiety, amelioration of opiate, nicotine and/or ethanol addiction, traumatic brain injury, spinal cord injury, post-traumatic stress syndrome and/or Huntington's chorea, in a patient in need thereof, comprising administering to the patient:
a pharmaceutically effective amount of GLYX-13: 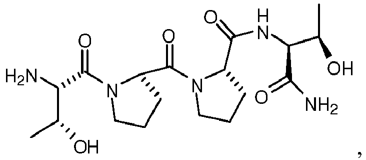 or pharmaceutically acceptable salt thereof, or
a pharmaceutically effective amount of a compound represented by Formula **I**: 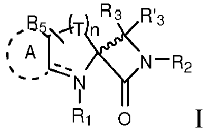 and pharmaceutically acceptable salts, stereoisomers, and N-oxides thereof; wherein
T is, independently for each occurrence, CR₄R₄', and n is 0, 1, 2 or 3;
A is optionally present and is selected from phenyl or pyridine, wherein A is optionally substituted by one or more substituents selected from Rₐ;
R₁ is selected from the group consisting of H, hydroxyl, -S(O)₂-C₁-₄alkyl; -SO₂, C₁-₄alkyl, C₂-C₄alkenyl, phenyl, R₇, or 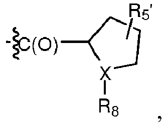 wherein C₁-₄alkyl, C₂-₄alkenyl, or phenyl is optionally substituted by one or more substituents selected from Rₐ;
X is CH or N;
R₃ and R₃ are independently selected from the group consisting of H, halogen, hydroxyl, phenyl, C₁-₄alkyl, amido, amine, or C₂-₄alkenyl, wherein C₁-₄alkyl, C₂-₄alkenyl and phenyl are optionally substituted by one or more substituents selected from Rₐ;
R₄ and R₄ are independently selected from the group consisting of H, halogen, hydroxyl, phenyl, C₁-₄alkyl, amido, amine, C₁-₄alkoxy or C₂-₄alkenyl, wherein C₁-₄alkyl, C₂-₄alkenyl, C₁-₄alkoxy, and phenyl are optionally substituted by one or more substituents selected from Rₐ;
R₂ is selected from the group consisting of H, R₇, -S(O)₂, S(O)₂-C₁-C₄alkyl, C₁-₄alkyl, hydroxyl, or phenyl wherein C₁-₄alkyl, C₂₋₄alkenyl and phenyl are optionally substituted by one or more substituents selected from Rₐ;
R₅ and R₅' are each independently selected from group consisting of H, halogen, C₁-₄alkyl, C₁-₄alkoxy, C₂-₄alkenyl, cyano, amino, phenyl, and hydroxyl, wherein C₁-₄alkyl, C₂-₄alkenyl and phenyl are optionally substituted by one or more substituents selected from Rₐ;
R₇ is selected from group consisting of -C(O)-R₉, -C(O)-O-R₉, or -C(O)-NR_{d}-R₉,
R₉ is selected from the group consisting of H, C₁-₄alkyl, phenyl, or heterocyclic, wherein C₁-₄alkyl, phenyl or heterocyclic is optionally substituted by 1, 2 or 3 substituents selected from R_{b}
R₈ is selected from group consisting of H, -C(O)- C₁-₄alkyl or C(O)-O-C₁-₄ alkyl, wherein C₁-₄alkyl is optionally substituted by 1, 2 or 3 substituents selected from Rₐ;
Rₐ is selected, independently for each occurrence, from carboxy, hydroxyl, halogen, amino, phenyl, C₁-₄alkyl, and C₁-₄alkoxy;
R_{b} is selected, independently for each occurrence, from the group consisting of carboxy, hydroxyl, halogen, amino, phenyl, C₁-₄alkyl, C₁-₄alkoxy, and -NH-R_{c};
R_{c} is selected, independently for each occurrence from the group consisting of: -C(O)-O-C₁-₄alkyl; and -C(O)-C₁-₄alkyl; and
R_{d} is selected, independently for each occurrence, H and C₁-₄alkyl;
and pharmaceutically acceptable salts, N-oxides or stereoisomers thereof.

13. A method of treating Alzheimer's disease, or memory loss that accompanies early stage Alzheimer's disease in a patient in need thereof, comprising administering to the patient:
a pharmaceutically effective amount of GLYX-13: 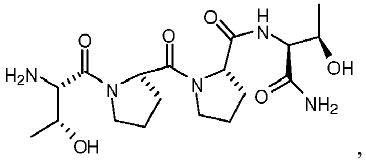 or pharmaceutically acceptable salt thereof, or
a pharmaceutically effective amount of a compound represented by Formula **I:** 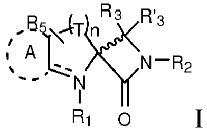 and pharmaceutically acceptable salts, stereoisomers, and N-oxides thereof; wherein
T is, independently for each occurrence, CR₄R₄', and n is 0, 1, 2 or 3;
A is optionally present and is selected from phenyl or pyridine, wherein A is optionally substituted by one or more substituents selected from Rₐ;
R₁ is selected from the group consisting of H, hydroxyl, -S(O)₂-C₁-₄alkyl; -SO₂, C₁-₄alkyl, C₂-C₄alkenyl, phenyl, R₇, or 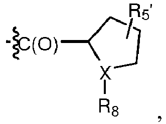 wherein C₁-₄alkyl, C₂-₄alkenyl, or phenyl is optionally substituted by one or more substituents selected from Rₐ;
X is CH or N;
R₃ and R₃ are independently selected from the group consisting of H, halogen, hydroxyl, phenyl, C₁-₄alkyl, amido, amine, or C₂-₄alkenyl, wherein C₁-₄alkyl, C₂-₄alkenyl and phenyl are optionally substituted by one or more substituents selected from Ra;
R₄ and R₄ are independently selected from the group consisting of H, halogen, hydroxyl, phenyl, C₁-₄alkyl, amido, amine, C₁-₄alkoxy or C₂-₄alkenyl, wherein C₁-₄alkyl, C₂-₄alkenyl, C₁-₄alkoxy, and phenyl are optionally substituted by one or more substituents selected from Rₐ;
R₂ is selected from the group consisting of H, R₇, -S(O)₂, S(O)₂-C₁-C₄alkyl, C₁-₄alkyl, hydroxyl, or phenyl wherein C₁-₄alkyl, C₂₋₄alkenyl and phenyl are optionally substituted by one or more substituents selected from Rₐ;
R₅ and R₅' are each independently selected from group consisting of H, halogen, C₁-₄alkyl, C₁-₄alkoxy, C₂-₄alkenyl, cyano, amino, phenyl, and hydroxyl, wherein C₁-₄alkyl, C₂-₄alkenyl and phenyl are optionally substituted by one or more substituents selected from Rₐ;
R₇ is selected from group consisting of -C(O)-R₉, -C(O)-O-R₉, or -C(O)-NR_{d}-R₉,
R₉ is selected from the group consisting of H, C₁-₄alkyl, phenyl, or heterocyclic, wherein C₁-₄alkyl, phenyl or heterocyclic is optionally substituted by 1, 2 or 3 substituents selected from R_{b}
R₈ is selected from group consisting of H, -C(O)- C₁-₄alkyl or C(O)-O-C₁-₄ alkyl, wherein C₁-₄alkyl is optionally substituted by 1, 2 or 3 substituents selected from Rₐ;
Rₐ is selected, independently for each occurrence, from carboxy, hydroxyl, halogen, amino, phenyl, C₁-₄alkyl, and C₁-₄alkoxy;
R_{b} is selected, independently for each occurrence, from the group consisting of carboxy, hydroxyl, halogen, amino, phenyl, C₁-₄alkyl, C₁-₄alkoxy, and -NH-R_{c};
R_{c} is selected, independently for each occurrence from the group consisting of: -C(O)-O-C₁-₄alkyl; and -C(O)-C₁-₄alkyl; and
R_{d} is selected, independently for each occurrence, H and C₁-₄alkyl;
and pharmaceutically acceptable salts, N-oxides or stereoisomers thereof.

14. A method of treating Huntington's disease, in a patient in need thereof, comprising administering to the patient:
a pharmaceutically effective amount of GLYX-13: 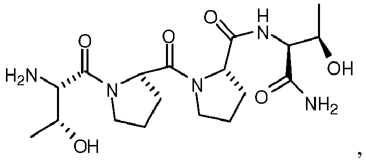 or pharmaceutically acceptable salt thereof, or
a pharmaceutically effective amount of a compound represented by Formula **I**: 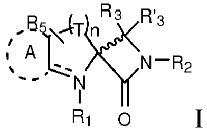 and pharmaceutically acceptable salts, stereoisomers, and N-oxides thereof; wherein
T is, independently for each occurrence, CR₄R₄', and n is 0, 1, 2 or 3;
A is optionally present and is selected from phenyl or pyridine, wherein A is optionally substituted by one or more substituents selected from Rₐ;
R₁ is selected from the group consisting of H, hydroxyl, -S(O)₂-C₁-₄alkyl; -SO₂, C₁-₄alkyl, C₂-C₄alkenyl, phenyl, R₇, or 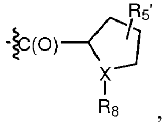 wherein C₁-₄alkyl, C₂-₄alkenyl, or phenyl is optionally substituted by one or more substituents selected from Rₐ;
X is CH or N;
R₃ and R₃ are independently selected from the group consisting of H, halogen, hydroxyl, phenyl, C₁-₄alkyl, amido, amine, or C₂-₄alkenyl, wherein C₁-₄alkyl, C₂-₄alkenyl and phenyl are optionally substituted by one or more substituents selected from Rₐ;
R₄ and R₄ are independently selected from the group consisting of H, halogen, hydroxyl, phenyl, C₁-₄alkyl, amido, amine, C₁-₄alkoxy or C₂-₄alkenyl, wherein C₁-₄alkyl, C₂-₄alkenyl, C₁-₄alkoxy, and phenyl are optionally substituted by one or more substituents selected from Rₐ;
R₂ is selected from the group consisting of H, R₇, -S(O)₂, S(O)₂-C₁-C₄alkyl, C₁-₄alkyl, hydroxyl, or phenyl wherein C₁-₄alkyl, C₂₋₄alkenyl and phenyl are optionally substituted by one or more substituents selected from Rₐ;
R₅ and R₅' are each independently selected from group consisting of H, halogen, C₁-₄alkyl, C₁-₄alkoxy, C₂-₄alkenyl, cyano, amino, phenyl, and hydroxyl, wherein C₁-₄alkyl, C₂-₄alkenyl and phenyl are optionally substituted by one or more substituents selected from Rₐ;
R₇ is selected from group consisting of -C(O)-R₉, -C(O)-O-R₉, or -C(O)-NR_{d}-R₉,
R₉ is selected from the group consisting of H, C₁-₄alkyl, phenyl, or heterocyclic, wherein C₁-₄alkyl, phenyl or heterocyclic is optionally substituted by 1, 2 or 3 substituents selected from R_{b}
R₈ is selected from group consisting of H, -C(O)- C₁-₄alkyl or C(O)-O-C₁-₄ alkyl, wherein C₁-₄alkyl is optionally substituted by 1, 2 or 3 substituents selected from Rₐ;
Rₐ is selected, independently for each occurrence, from carboxy, hydroxyl, halogen, amino, phenyl, C₁-₄alkyl, and C₁-₄alkoxy;
R_{b} is selected, independently for each occurrence, from the group consisting of carboxy, hydroxyl, halogen, amino, phenyl, C₁-₄alkyl, C₁-₄alkoxy, and -NH-R_{c};
R_{c} is selected, independently for each occurrence from the group consisting of: -C(O)-O-C₁-₄alkyl; and -C(O)-C₁-₄alkyl; and
R_{d} is selected, independently for each occurrence, H and C₁-₄alkyl;
and pharmaceutically acceptable salts, N-oxides or stereoisomers thereof.


SEQUENCE LISTING

<110> North Western University

<120> METHODS OF TREATING ALZHEIMER'S DISEASE, HUNTINGTON'S DISEASE,
AUTISM, OR OTHER DISORDERS

<130> P59079EP-K

<140> EP12777016.2
<141> 2012-04-27

<150> US61/479593
<151> 2011-04-27

<150> US61/527744
<151> 2011-07-26

<160> 1

<170> PatentIn version 3.5

<210> 1
<211> 4
<212> PRT
<213> Artificial Sequence

<220>
<223> SEQ ID NO: 1

<400> 1

Thr Pro Pro Thr
1